# EUROPEAN PATENT APPLICATION

(11) **EP 1 731 139 A1**
(43) Date of publication of application: **13.12.2006**
(21) Application number: 05728929.0
(22) Date of filing: 31.03.2005
(51) Int. Cl.: A61K 9/107, A61K 31/196, A61K 31/405, A61K 45/00, A61K 47/14, A61K 47/26, A61K 47/34, A61K 47/42, A61K 47/44, A61P 29/00

(54) **S/O TYPE PHARMACEUTICAL PREPARATION AND PROCESS FOR PRODUCING THE SAME**

(30) Priority: 31.03.2004 JP 2004103347
(71) Applicant: ASPION CO., LTD., Tokushima-shi, Tokushima 7700815 (JP)
(72) Inventor: GOTO, Masahiro, Fukuoka-shi, Fukuoka 8190044 (JP); KAMIYA, Noriho, Fukuoka-shi, Fukuoka 8190002 (JP); HIRATA, Akihiko, Tokushima-shi, Tokushima 770-0872 (JP); FUJII, Takeru, Naruto-shi, Tokushima 7720051 (JP)
(74) Representative: Gille Hrabal Struck Neidlein Prop Roos
(86) International application number: PCT/JP2005/006812
(87) International publication number: WO 2005/094789

(57) **Abstract**

The present invention provides a pharmaceutical preparation that significantly reduces leakage of a low-molecule medicine in a strong acidic environment, while allowing release of the low-molecule medicine in the enteric canal or the like which is in a weak acidic to neutral environment. The S/O type pharmaceutical preparation of the present invention is **characterized in** comprising a medicine-containing complex dissolved or dispersed in an oil phase, wherein the complex contains a mixture and a surfactant, the mixture is covered by the surfactant, and the mixture contains a hydrophilic low molecule medicine, and a hydrophilic medicine-leakage-suppressive protein and/or a medicine-leakage-suppressive polysaccharide.

## Description

### TECHNICAL FIELD

The present invention relates to an S/O type pharmaceutical preparation and a method for producing the same.

### BACKGROUND ART

In order that an orally administered medicine may be absorbed through gastrointestinal tracts such as stomach or small intestine, the medicine must be dissolved in a site for absorption. Accordingly, in case of a medicine having poor solubility in gastrointestinal tracts, so-called hardly-soluble medicine, various drug preparation techniques such as formation of salt and use of surfactant are employed to improve the solubility of such a medicine in gastrointestinal tracts and improve the digestibility.

On the other hand, however, the solubility in gastrointestinal tracts sometimes becomes a problem. For example, some non-steroidal anti-inflammation drugs (hereinafter, referred to as "NSAID") inhibit cyclooxygenase I which is an enzyme exhibiting gastric mucosa protecting activity, and induce gastric ulcer. In particular, NSAID such as diclofenac sodium which is a carboxylic acid compound is protonated by gastric acid in the stomach to become non-ionic, and thus its lipophilicity is increased. As a result, the membrane transmissivity is increased and the NSAID accumulates in stomach tissues, to cause damages in the gastric mucosa.

Therefore, in oral administration of such a medicine, it is necessary to realize a pharmaceutical preparation capable of releasing a medicine in the stage from duodenum to small intestine but not in the stomach.

In this respect, inventors of the present invention have developed an S/O/W type pharmaceutical preparation which is prepared by dispersing a lyophiled mixture of an aqueous solution of enzyme, bioactive peptide or medicine and an organic solvent solution of surfactant (S/O type pharmaceutical preparation, Solid in Oil) in an oil phase, and dispersing the resultant oil phase in an aqueous phase (Japanese Unexamined Patent Publication No. 2004-43355; S. Okazaki, N. Kamiya, K. Abe, M. Goto, F. Nakashio, Biotechnol. Bioeng., 55(2), pp.455-460 (1997); N. Kamiya, S. Okazaki, M. Goto, Biotechnol. Tech., 11(6), pp. 375-378 (1997)). For example, in Example of the Publication No. 2004-43355, an S/O/W type pharmaceutical preparation containing insulin or irinotecan hydrochloride as a medicine is prepared. The experimental data showing suppression of leakage to the aqueous phase is also described. Therefore, the pharmaceutical preparation seems to have a certain degree of sustained releasability.

In addition to the above, Japanese Unexamined Patent Publication No. 6-303973 describes an enzyme composition covered with surfactant, which includes a surfactant, enzyme, water and salts, and has high activity even in an water-insoluble organic solvent. PCT International Application Japanese Translation No. 2003-501404 discloses a pharmaceutical preparation in which solid phase particles consisting of dehydrated products of a medicine, a surfactant, and a membrane permeation promoter are suspended in a delivery medium. The pharmaceutical preparation allows a therapeutic protein or peptide to be absorbed transmucosally. Furthermore, PCT International Application Japanese Translation No. 10-510256 No. 10-510256 discloses a method for producing a hydrophobic preparation, in which a hydrophobic solvent is supplied around a hydrophilic substance covered with an amphiphilic substance. This technique is intended for solubilizing the hydrophilic molecule in the hydrophobic phase, and in Example, not only aprotinin which is a peptide but also a low-molecule ascorbic acid is used as the hydrophilic molecule.

### DISCLOSURE OF THE INVENTION

As described above, an S/O type pharmaceutical preparation wherein a solid phase containing a hydrophilic medicine is dispersed in an oil phase has been conventionally known.

However, as a result of further studies for such techniques, the inventors of the present invention found that a medicine tends to leak when a low-molecular compound is blended as a medicine in the S/O type pharmaceutical preparation. For example, in an example of Japanese Unexamined Patent Publication No. 2004-43355, experimental data is disclosed that leakage of a medicine into aqueous solution is suppressed by an S/O/W type pharmaceutical preparation containing irinotecan hydrochloride. However, irinotecan which is a basic compound is thought to be more likely to leak in the stomach which is in a strong acidic environment. Also, other medicines having higher solubility to strong-acidic gastric acid than to neutral water are known. On the other hand, when an S/O type pharmaceutical preparation is used as an oral preparation, it is necessary to increase the medicine releasability in the enteric canal which is in a weak acidic to neutral environment.

Accordingly, it is an object of the present invention to provide a pharmaceutical preparation which can release a low-molecule medicine when the preparation comes into contact with the enteric canal or conjunctiva, or a body fluid, blood and the like exuded in surface of a wound skin in a weak acidic to neutral environment, while significantly reduces leakage of a low-molecule medicine in a strong acidic environment.

In order to solve the aforementioned problems, inventors of the present invention diligently studied about ingredients of an S/O type pharmaceutical preparation. It was finally found that combination of a specific protein and polysaccharide allows rapid release of a low-molecule medicine in an artificial intestinal juice, with little leakage of the same in an artificial gastric juice, and the inventors accomplished the present invention.

An S/O type pharmaceutical preparation of the present invention comprises a medicine-containing complex dissolved or dispersed in an oil phase, wherein the complex contains a mixture and a surfactant, the mixture is covered by the surfactant, and the mixture contains a hydrophilic low molecule medicine, and a hydrophilic medicine-leakage-suppressive protein and/or a medicine-leakage-suppressive polysaccharide.

Further, an S/O/W type pharmaceutical preparation of the present invention is characterized in that the S/O type pharmaceutical preparation is dispersed in an aqueous phase.

The above mentioned S/O type pharmaceutical preparation can be prepared by a method, comprising the steps of: mixing a water-based solution containing a hydrophilic low-molecule medicine and a hydrophilic medicine-leakage-suppressive protein and/or a medicine-leakage-suppressive polysaccharide with an organic solvent solution containing a surfactant to prepare a W/O type emulsion; lyophilizing the W/O type emulsion to prepare a medicine-containing complex; and dissolving or dispersing the medicine-containing complex in an oil phase.

### BRIEF DESCRIPTION OF THE DRAWINGS

Fig. 1 shows results of elution test of diclofenac sodium from the pharmaceutical preparation of the present invention in artificial gastric juice and in artificial intestine juice, wherein (A) shows a result for the preparation containing bovine serum albumin (BSA) as a medicine-leakage-suppressive protein, (B) shows a result for the preparation containing ovalbumin (OVA), and (C) shows a result for the preparation containing casein.
Fig. 2 shows a result of elution test of diclofenac sodium from an S/O type pharmaceutical preparation not containing a medicine-leakage-suppressive protein in artificial gastric juice and in artificial intestine juice.
Fig. 3 shows conditions of gastric mucosa of rats after administration of the pharmaceutical preparation of the present invention or a control, wherein (A) shows the stomach after administration of a pharmaceutical preparation of No.10 in Test example 2 as will be described later, and (B) shows the stomach after administration of No.5 (diclofenac sodium solution) in which the black part is a bleeding site.
Fig. 4 shows results of stability test for the pharmaceutical preparation of the present invention in a severe condition, wherein
   (A) shows a test result of drug release into an artificial gastric juice at one, two and three months after starting the test, and
   (B) shows a test result of drug release into an artificial intestine juice.
Fig. 5 is a graph showing temporal change of blood concentration in beagle dogs when the pharmaceutical preparation of the present invention containing diclofenac sodium is administered (-◆-), or a simple solution of diclofenac sodium (- -◇- -).
Fig. 6 shows results of drug release of the pharmaceutical preparation of the present invention in artificial gastric juice
   (A) and in artificial intestine juice (B), wherein (-◆-) shows the result for the preparation containing LM pectin, (-▲-) shows the result for the preparation containing HM pectin, and (-■-) shows the result for the preparation containing hydroxypropylmethyl cellulose phthalate, as a medicine-leakage-suppressive polysaccharide.

### BEST MODE FOR CARRYING OUT THE INVENTION

The S/O type pharmaceutical preparation of the present invention is characterized in comprising a medicine-containing complex dissolved or dispersed in an oil phase, wherein the complex contains a mixture and a surfactant, the mixture is covered by the surfactant, and the mixture contains a hydrophilic low molecule medicine, and a hydrophilic medicine-leakage-suppressive protein and/or a medicine-leakage-suppressive polysaccharide.

First, a medicine-containing complex constituting the S/O type pharmaceutical preparation of the present invention will be explained.

The medicine-containing complex is mainly composed of a hydrophilic low-molecule medicine, and a hydrophilic medicine-leakage-suppressive protein and/or a medicine-leakage-suppressive polysaccharide (hereinafter sometimes referred to as "medicine-leakage-suppressive component"), and a surfactant. Hydrophilic part of the surfactant associates a hydrophilic mixture of the medicine and the medicine-leakage-suppressive component, and the surfactant envelopes the hydrophilic mixture. The complex may comprise exclusively a hydrophilic low-molecule medicine, a medicine-leakage-suppressive component, and a surfactant so as to prevent the particles from becoming too large, or may include a pharmaceutically acceptable pharmaceutical component.

A hydrophilic medicine is used in the present invention, since a hydrophobic medicine may fail to form an S/O type preparation. Also, a low-molecule medicine is used due to the fact that the smaller the molecular weight of the medicine is, the larger the medicine leakage amount in the stomach is, and the object to be solved of the present invention lies on this point. The hydrophilic low-molecule medicine of the present invention has a molecular weight of not more than 10,000, more preferably 5,000 or less, and further more preferably 1,000 or less.

As the hydrophilic low-molecule medicine, a medicine that directly inflicts damage to the stomach is conceivable. Since the pharmaceutical preparation of the present invention can significantly suppress leakage of the medicine in the stomach, the preparation can cover the drawbacks accompanying such a medicine. As such a medicine, NSAID having an inhibitory activity of cyclooxygenase I involved in protection of gastric mucosa can be exemplified. For example, one or more than one selected from a group consisting of diclofenac, indomethacin, and salts thereof are conceivable.

The hydrophilic medicine-leakage-suppressive protein and the medicine-leakage-suppressive polysaccharide in the present invention have an activity to suppress leakage of the medicine of the present invention in the stomach, and any types of substances may be applied as far as they are acceptable as ingredients of pharmaceutical preparation. In order to achieve effective medicine-leakage-suppressive activity, the component having a molecular weight of 10,000 or more is preferably used.

Examples of the medicine-leakage-suppressive protein include serum albumin (molecular weight: about 67,000), ovalbumin (molecular weight: about 45,000), casein (molecular weight: about 19,000 or more), lysozyme (molecular weight: about 14,000 or more), and lipase (molecular weight: about 45,000) . One of these may be selected or more than one may be selected and combined for used. Preferably, one or more than one selected from a group consisting of serum albumin, ovalbumin, and casein may be used.

As the medicine-leakage-suppressive polysaccharide, for example, LM pectin, HM pectin, hydroxypropylmethyl cellulose phthalate, heparin, alginic acid, and carboxymethyl cellulose can be exemplified, and one of these may be selected or more than one may be selected and combined for use. Preferably, one or more than one selected from a group consisting of LM pectin, HM pectin and hydroxypropylmethyl cellulose phthalate may be used.

It is to be noted that the molecular weight of medicine-leakage-suppressive protein or medicine-leakage-suppressive polysaccharide varies depending on its origin and the like. For example, while pectin generally has a molecular weight ranging from 50,000 to 150,000, the pectin used in the example described later has a molecular weight ranging from 20, 000 to 40, 000. However, the molecular weight of the components exemplified in the above is generally 10,000 or more.

The surfactant is not particularly limited insofar as it is pharmaceutically acceptable. For example, non-ionic surfactant, negative-ionic surfactant, positive-ionic surfactant, ampholytic surfactant, and bile salt can be exemplified.

Examples of the non-ionic surfactant include polyglycerin condensed ricinolein acid ester, decaglycerin ester, glycerin fatty acid ester, polyglycerin fatty acid ester, polyoxyethylene glycerin fatty acid ester, sorbitan fatty acid ester, polyoxyethylene sorbit fatty acid ester, polyoxyethylene castor oil/hardened castor oil, sucrose fatty acid ester such as sucrose stearic acid ester, sucrose palmitic acid ester, sucrose myristic acid ester, sucrose oleic acid ester, sucrose lauric acid ester, sucrose erucic acid ester, sucrose mixed fatty acid ester, and the like. One of these may be selected or more than one may be selected and combined for use.

As such a non-ionic surfactant, ester compounds based on unsaturated fatty acids such as erucic acid or oleic acid are preferred, and sucrose erucic acid ester, sucrose oleic acid ester, and sucrose mixed fatty acid ester are more preferred. Alternatively, one or two or more selected from a group consisting of glycerin fatty acid ester, polyglycerinfatty acid ester, polyoxyethylene glycerin fatty acid ester, sorbitan fatty acid ester, sucrose fatty acid ester, polyoxyethylene sorbit fatty acid ester, polyoxyethylene castor oil, and hardened castor oil may be used.

As the surfactant, a highly hydrophobic surfactant such as that having a HLB value of 10 or less is preferably used, since such a surfactant facilitates dissolution and dispersion of the medicine-containing complex in an oil phase.

In the medicine-containing complex, the weight ratio of medicine-leakage-suppressive component relative to the hydrophilic low-molecule medicine is preferably in the range of 0.01 to 100. If the weight ratio is less than 0.01, the medicine leakage suppressing ability in the stomach may not be sufficiently exerted in some cases, whereas if the weight ratio is more than 100, the medicine amount in the complex is small so that the effect of the medicine itself may not be exerted in some cases. More preferably, the weight ratio is in the range of 0.1 to 10, and further more preferably in the range of 0.5 to 5.

Further, the weight ratio of surfactant with respect to the hydrophilic low-molecule medicine and the medicine-leakage-suppressive component is preferably in the range of 0.5 to 100, more preferably in the range of 1 to 50, and further more preferably in the range of 2 to 25.

The S/O type pharmaceutical preparation of the present invention is a solution or suspension in which the above medicine-containing complex is dissolved or dispersed in an oil phase. Whether the preparation is a solution or a suspension depends on the type and amount of surfactant and oil phase, and with or without treatment of sonication and the like.

The oil phase used in the present invention is not particularly limited insofar as it is pharmaceutically acceptable, and examples include vegetable oils, animal oil, neutral lipids such as mono-substituted, di-substituted, or tri-substituted glyceride, synthetic oil, and sterol derivatives.

Specific examples include cooking oils such as soybean oil, cotton oil, rape oil, sesame oil, corn oil, peanut oil, safflower oil, sunflower oil, olive oil, canola oil, and perilla oil; animal oils such as beef fat, pork oil, and fish oil; neutral lipids such as triolein, trilinolein, tripalmitin, tristearin, trimyristin, and triarachidonin; synthetic lipids such as azone; and sterol derivatives such as cholesteryl oleate, cholesteryl linoleate, cholesteryl myristate, cholesteryl parmitate, and cholesteryl arachidate. One of these may be selected or more than one may be selected and combined for use. Preferably, one or more than one selected from a group consisting of soybean oil, sesame oil, olive oil, safflower oil, sunflower oil, canola oil, and perilla oil is used. Particularly preferred is to use triglyceride or cooking oils based on the same, and practically preferred is soybean oil, especially highly-purified soybean oil.

The oil phase is degraded by lipase existing in the enteric canal. Therefore, the S/O type pharmaceutical preparation of the present invention little disintegrates in the stomach and reaches the enteric canal where the preparation disintegrates to become able to release the medicine.

The ratio of the oil phase in the S/O type pharmaceutical preparation of the present invention varies depending on the type of oil component or other constituents, and the ratio is preferably in the range of 50 to 99.5 w/v%, and preferably in the range of 60 to 90w/v%.

The S/O/W type pharmaceutical preparation of the present invention is a dispersion of the above mentioned S/O type pharmaceutical preparation in an aqueous phase. Such a pharmaceutical preparation is easily administered and has great convenience.

The kind of aqueous phase is not particularly limited insofar as it is pharmaceutically acceptable, and for example, pure water, purified water, distilled water, saline, buffer, and the like may be used.

The S/O type pharmaceutical preparation of the present invention may be produced by a method comprising the steps of: (1) mixing a water-based solution containing the hydrophilic low-molecule medicine and the hydrophilic medicine-leakage-suppressive component with an organic solvent solution containing the surfactant to prepare a W/O type emulsion; (2) lyophilizing the W/O type emulsion to prepare a medicine-containing complex; and (3) dissolving or dispersing the medicine-containing complex in an oil phase.

### (1) W/O type emulsion preparing step

In this step, first, an aqueous solution of the low-molecule medicine and the medicine-leakage-suppressive component is prepared. Examples of water used here include pure water, purified water, distilled water, saline, buffer, and the like. Water miscible organic solvent such as ethanol may be added as necessary.

The concentration of the low-molecule medicine and the medicine-leakage-suppressive component in the aqueous solution is especially not limited insofar as the ingredients are substantially fully dissolved, and may be, for example, about 5 to 30mg/mL.

Separately, an organic solvent solution of the surfactant is prepared. The organic solvent is not particularly limited insofar as it is able to dissolve the surfactant and removable by diffusion in the subsequent step, and examples of such solvent include alcohols such as methanol and ethanol; aliphatic hydrocarbons such as hexane; and aromatic hydrocarbons such as toluene. The concentration is not particularly limited, and may be, for example, about 1 to 10 % by mass.

Next, the aqueous solution of the low-molecule medicine and the medicine-leakage-suppressive component is mixed with the organic solution of the surfactant, and a W/O type emulsion is prepared according to a conventional method. For example, high speed stirring using homogenizer, stirring by a stirrer such as propeller mixer or disper, and additionally irradiation of ultrasonic wave may be employed.

### (2) Lyophilization step

In this step, the W/O type emulsion obtained in the above step (1) is lyophiled to give a medicine-containing complex. As to specific conditions, a conventional method is employed. In this step, it is preferred to remove the water and the organic solvent substantially entirely. This is because the water may cause medicine leakage in the stomach, and the organic solvent may adversely affect on the living body. To be more specific, the moisture content should be about 1% or less measured by the Karl Fischer method.

### (3) Dispersing step

In this step, the medicine-containing complex obtained in the step (2) is dissolved or dispersed in an oil phase, to thereby produce an S/O type pharmaceutical preparation. More specifically, as is the case in the step (1), high speed stirring using homogenizer, stirring by a stirrer such as propeller mixer or disper, and additionally irradiation of ultrasonic wave may be employed.

The amount of oil phase used in this step is, for example, about 1 to 10 mL per 1g of the medicine-containing complex although it depends on the kind and affinity of the surfactant and the oil phase, or the like.

The obtained S/O type pharmaceutical preparation may further be dispersed in an aqueous phase according to a conventional method to give an S/O/W type pharmaceutical preparation.

The S/O type pharmaceutical preparation and the S/O/W type pharmaceutical preparation according to the present invention little release the medicine in the stomach, whereas its particles disintegrate to allow release of the medicine in an enteric canal. Therefore, the preparation is very excellent as an oral drug delivery system for medicines that inflict direct damage to the stomach and for medicines that are instable in a strong acidic condition. Also, the S/O type pharmaceutical preparation and the S/O/W type pharmaceutical preparation of the present invention are excellent in stability.

A dosage of the present pharmaceutical preparation may be adjusted depending on the kind and amount of blended medicine.

In the following, Examples and Test examples are given and the present invention is more specifically explained. However, it is to be noted that the scope of the present invention is not limited by these examples.

### Examples

### Production example 1 Production of S/O type complex suspension of the present invention

To 10 mL of 10mM phosphate buffer (pH 8.0), diclofenac sodium (hereinafter referred to as "DFNa"), and bovine serum albumin (BSA, molecular weight: 67,000 or more), ovalbumin (OVA, molecular weight: 95, 000 or more) or casein (molecular weight: 19, 000 or more) as a medicine-leakage-suppressive protein was dissolved, respectively at a concentration of 10 mg/mL. To each solution, 20 mL of 5 wt% solution of sucrose erucic acid ester (manufactured by Mitsubishi-Kagaku Foods Corporation, ER-290, erucic acid 90 wt%, HLB: 2) in toluene was added. The mixture was stirred at high speed (26, 000rpm) by a homogenizer, to prepare a W/O type emulsion. The emulsion was lyophiled for a day, to prepare a surfactant-DFNa complex containing a medicine-leakage-suppressive protein. To each of these complexes, 5 mL of soybean oil was added, followed by dispersion by irradiation of ultrasonic waves, to give an S/O type complex suspension of the present invention.

### Comparative production example 1

A surfactant-DFNa complex not containing medicine-leakage-suppressive protein was prepared in the same manner as Production example 1 described above except that medicine-leakage-suppressive protein was not blended. An S/O type complex suspension was obtained in the same manner as the above Production example 1 by using the complex.

### Test example 1 Drug release test

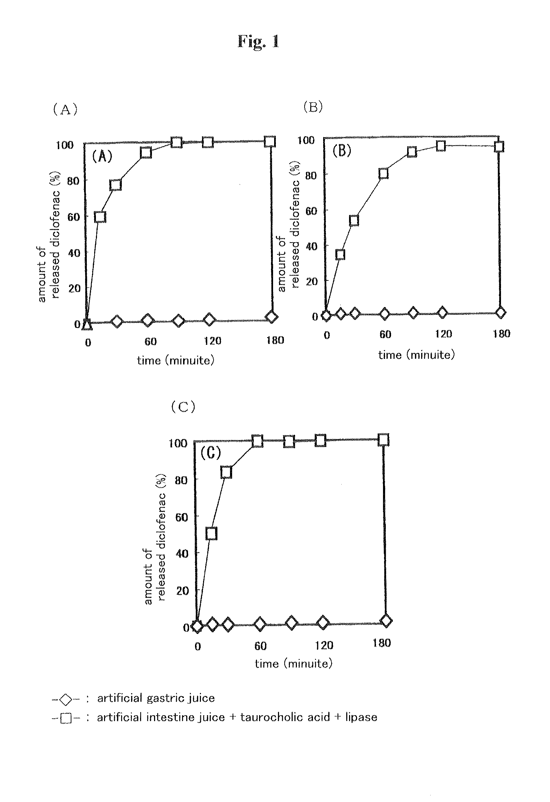

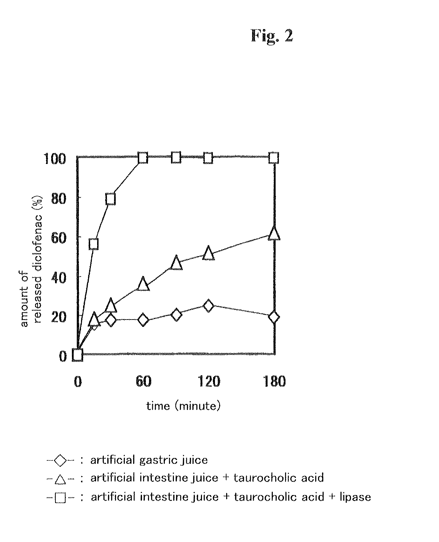

To 10mL of artificial gastric juice (The Japanese Pharmacopoeia, Disintegration test first fluid, pH 1.2) or 10mL of artificial intestine juice (The Japanese Pharmacopoeia, Disintegration test second fluid, pH 6.8), taurocholic acid (20 mM) and lipase (500 U/mL) were added. To the mixture under stirring at 37 °C, 100 mg of the S/O type complex solution obtained in the above Production example 1 or Comparative production example 1 was added. Then samples collected with time were subjected to centrifugal ultrafiltration (2900 rpm, 3 minutes), and analyzed by HPLC (270nm) to determine percentage of leaked DFNa to the entirety. Results are shown in Figs. 1 and 2.

As shown in Fig. 2, the S/O type complex suspension not containing medicine-leakage-suppressive protein immediately released DFNa in artificial intestine juice containing lipase, and suppressed release of DFNa in the artificial gastric juice to some extent.

On the other hand, as shown in Fig. 1, the S/O type complex suspension of the present invention seldom released DFNa in the artificial gastric juice, while immediately released DFNa in the artificial intestine juice. Accordingly, when the S/O type complex suspension of the present invention is orally administered, the medicine will be seldom released in the stomach and released in the intestine. Therefore, it is demonstrated that the S/O type complex suspension of the present invention is a very excellent drug delivery system.

### Test example 2 Gastric mucosa stimulus test

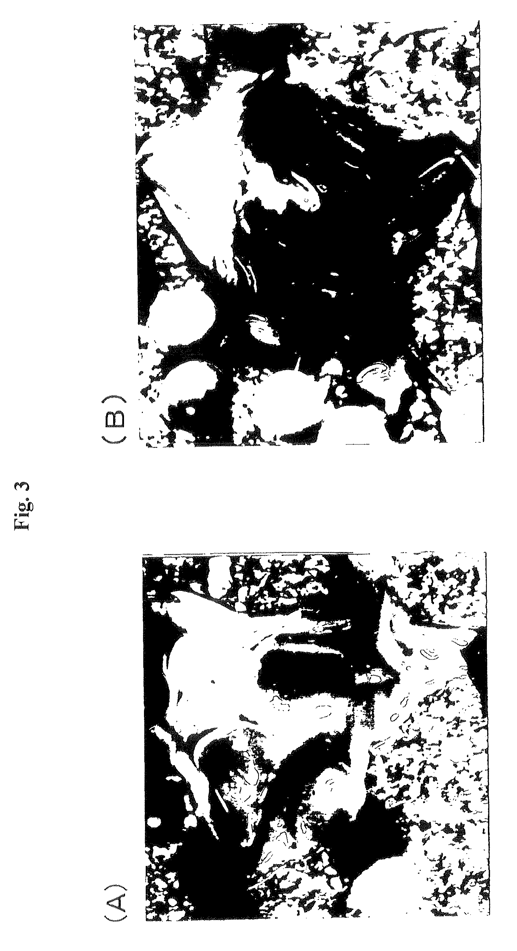

Gastric mucosa stimulus test for the S/O type complex suspension obtained in Production example 1 was carried out. Specifically, according to the method disclosed by Tsurumi et al. (Folia Pharmacol. Japan, 69, pp.319-334 (1973)), five male Wistar rats having an average body weight of 140g were fasted for 18 hours in advance, and were administered so that the dose amount of DFNa was 50 mg/kg body weight by means of an intrastomach catheter for rat. After 3. 5 hours, the rats were sacrificed and dissected, and the condition of the gastric mucosa was observed. Also blood collected after 3. 5 hours was centrifuged to obtain a plasma sample, and DFNa concentration in the plasma was determined by HPLC. As controls, a base excluding DFNa in Production example 1, saline, or DFNa solution in saline was administered, and a similar processing was carried out. The results are shown in Table 1 and Fig. 3.

**Table 1**

| No. | Administered formulation | DFNa dose amount (mg/kg body weight) | Plasma DFNa concentration | Condition of gastric mucosa |
|---|---|---|---|---|
| 1 | Base only | 0 | 0 | Not abnormal |
| 2 | Saline | 0 | 0 | Not abnormal |
| 3 | DFNa solution | 50 | 6.64 | Dotted bleeding |
| 4 | DFNa solution | 50 | 3.99 | Dotted bleeding |
| 5 | DFNa solution | 50 | 7.98 | Gastric ulcer |
| | | | Average: 6.21±2.03 | |
| 6 | Inventive pharmaceutical preparation | 50 | 8.67 | Slight dotted bleeding |
| 7 | Inventive pharmaceutical preparation | 50 | 7.13 | Dotted bleeding |
| 8 | Inventive pharmaceutical preparation | 50 | 4.35 | Slight dotted bleeding |
| 9 | Inventive pharmaceutical preparation | 50 | 5.13 | Not abnormal |
| 10 | Inventive pharmaceutical preparation | 50 | 6.33 | Not abnormal |
| | | | Average: 6.32±1.69 | |

A dose amount of DFNa in this test is 60 to 120 times the usual single dose concentration. Therefore, as is apparent from the results, clear abnormality is observed in gastric mucosa for the case where DFNa is simply dissolved in saline. On the other hand, for the case where the inventive pharmaceutical preparation is administered, abnormality in gastric mucosa is clearly reduced although DFNa in plasma are almost identical. This is attributed to the fact that the inventive pharmaceutical preparation will not impose a burden on stomach while favorable blood concentration is kept because the preparation will release DFNa in the intestine but little release DFNa in the stomach.

### Test example 3 Stability test

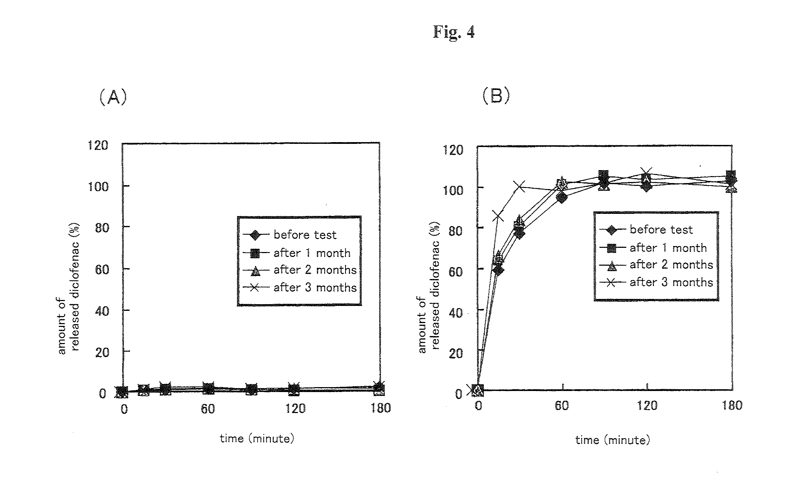

Stability of the inventive pharmaceutical preparation obtained in Production example 1 was tested in a severe environment at a test temperature of 40 °C and a relative humidity of 75 %. To be more specific, a drug release test which is similar to Test example 1 was carried out at one, two, and three months after starting the test, and change in characteristic was examined. Results are shown in Fig. 4.

As can be seen from these results, even after three-month stock in the severe condition, the inventive pharmaceutical preparation kept the characteristic that the preparation little releases DFNa in artificial gastric juice but rapidly releases in artificial intestine juice. The results demonstrate high stock stability.

### Test example 4 Blood concentration test

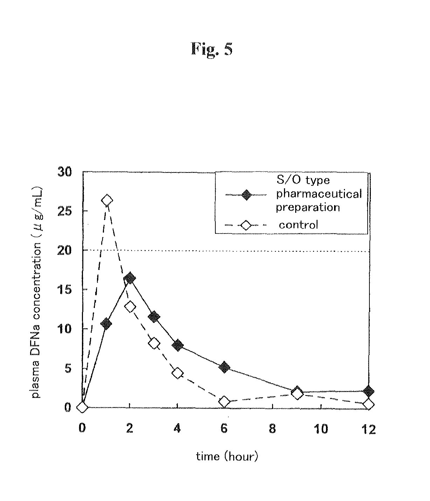

To a beagle dog fasted overnight, the inventive pharmaceutical preparation obtained in Production example 1 was orally administered so that DFNa dose concentration was 6 mg/kg body weight, and blood was collected with time. The blood concentration was measured in a similar manner as Test example 2. As a control, a solution in which DFNa is dissolved in saline was administered, and similar measurement was carried out. Results are shown in Table 2 and Fig. 5.

**[Table 2]**

| Inventive Control pharmaceutical preparation | | |
|---|---|---|
| Maximum blood concentration (µg/mL) | 26.4 | 16.5 |
| Maximum blood concentration reach time (hour) | 1 | 2 |
| Serum half-life (hour) | 0.98 | 1.9 |
| Area under medicine concentration curve (µg·hour/mL) | 62.8 | 73.7 |

According to the result, the blood concentration immediately decreases following acute increase directly after administration by the DFNa solution. Preparations exhibiting such dynamics have a problem that adverse effect is likely to occur and the efficacy does not last. In contrast, when the inventive pharmaceutical preparation is administered, the blood concentration gently changes and no rapid increase or decrease is observed, and serum half-life is extended. This is attributable to the fact that the inventive pharmaceutical preparation little release DFNa in the stomach, and gradually disintegrates in enteric canals and releases DFNa. Further, when the inventive pharmaceutical preparation is used, the area under medicine concentration curve (AUC) increases by more than a dozen percentages compared to the case where a simple solution is administered. As the above, the inventive pharmaceutical preparation shows excellent dynamics in the body.

### Production example 2 Preparation of S/O type complex suspension according to the present invention

An S/O type complex suspension according to the present invention was prepared by using LM pectin (molecular weight: 20, 000 to 40,000), HM pectin (molecular weight: 20,000 to 40,000), or hydroxypropylmethyl cellulose phthalate (HPMCP, molecular weight: 80,000 to 110,000) as a medicine-leakage-suppressive polysaccharide instead of the medicine-leakage-suppressive protein in the above Production example 1.

### Test example 5

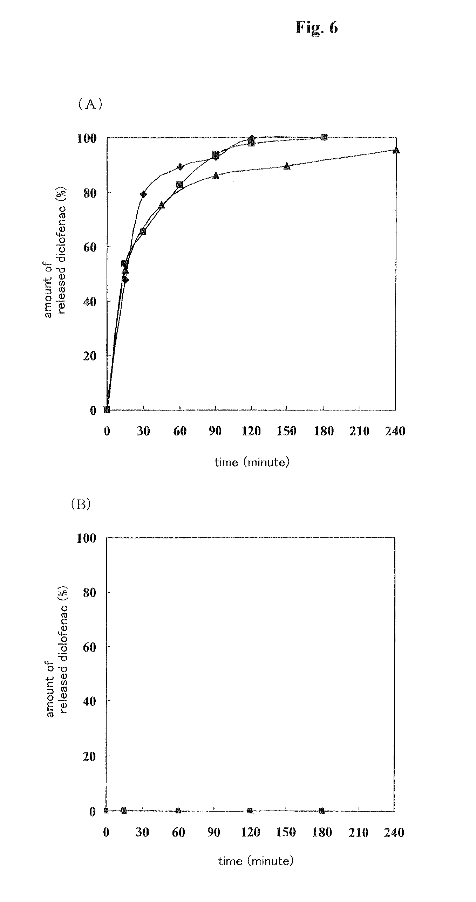

On the S/O type complex suspension obtained in Production example 2, a drug release test was carried out in the same manner as described in the above Test example 1. Results are shown in Fig. 6.

The results reveal that even when the medicine-leakage-suppressive polysaccharide is used instead of the medicine-leakage -suppressive protein, release of medicine in the stomach is suppressed, and release of medicine in the enteric canal is allowed.

### INDUSTRIAL APPLICABILITY

The S/O type pharmaceutical preparation of the present invention significantly suppresses leakage of a hydrophilic low-molecule medicine in the stomach. Therefore, even for a medicine that causes gastric ulcer like NSAID, the damage on the stomach is reduced. On the other hand, excellent drug releasability is realized in the small intestine, eye mucosa, blood or body fluid which is in a weak acidic to neutral environment. In addition, the S/O type pharmaceutical preparation of the present invention has excellent stability.

The method for producing an S/O type pharmaceutical preparation according to the present invention has excellent industrial availability because the method enables production of a pharmaceutical preparation having such good characteristics.

## Claims

1. An S/O type pharmaceutical preparation,
comprising a medicine-containing complex dissolved or dispersed in an oil phase,
wherein the complex contains a mixture and a surfactant, the mixture is covered by the surfactant, and
the mixture contains a hydrophilic low molecule medicine, and a hydrophilic medicine-leakage-suppressive protein and/or a medicine-leakage-suppressive polysaccharide.

2. The S/O type pharmaceutical preparation according to claim 1, wherein the hydrophilic medicine-leakage-suppressive protein and the medicine-leakage-suppressive polysaccharide have a molecular weight of not less than 10,000.

3. The S/O type pharmaceutical preparation according to claim 1 or 2, wherein the hydrophilic medicine-leakage-suppressive protein is one or more than one selected from a group consisting of serum albumin, ovalbumin, casein, lysozyme, lipase, colipase, and globulin.

4. The S/O type pharmaceutical preparation according to claim 1 or 2, wherein the hydrophilic medicine-leakage-suppressive protein is one or more than one selected from a group consisting of serum albumin, ovalbumin, and casein.

5. The S/O type pharmaceutical preparation according to claim 1. or 2, wherein the medicine-leakage-suppressive polysaccharide is one or more than one selected from a group consisting of LM pectin, HM pectin, hydroxypropylmethyl cellulose phthalate, heparin, alginic acid, and carboxymethyl cellulose.

6. The S/O type pharmaceutical preparation according to claim 1 or 2, wherein the medicine-leakage-suppressive polysaccharide is one or more than one selected from the group consisting of LM pectin, HM pectin, and hydroxypropylmethyl cellulose phthalate.

7. The S/O type pharmaceutical preparation according to any one of claims 1 to 6, wherein a weight ratio of the hydrophilic medicine- leakage-suppressive protein and/or the medicine-leakage-suppressive polysaccharide with respect to the low-molecule medicine is in a range of 0.5 to 5.

8. The S/O type pharmaceutical preparation according to any one of claims 1 to 7, wherein the oil phase is one or more than one selected from a group consisting of soybean oil, sesame oil, olive oil, safflower oil, sunflower oil, canola oil, and perilla oil.

9. The S/O type pharmaceutical preparation according to any one of claims 1 to 8, wherein the surfactant is a non-ionic surfactant.

10. The S/O type pharmaceutical preparation according to any one of claims 1 to 8, wherein the surfactant is one or more than one selected from a group consisting of glycerin fatty acid ester, polyglycerin fatty acid ester, polyoxyethylene glycerin fatty acid ester, sorbitan fatty acid ester, sucrose fatty acid ester, polyoxyethylene sorbit fatty acid ester, polyoxyethylene castor oil, and hardened castor oil.

11. The S/O type pharmaceutical preparation according to any one of claims 1 to 8, wherein the surfactant has a HLB value of not more than 10.

12. The S/O type pharmaceutical preparation according to any one of claims 1 to 11, wherein the low-molecule medicine is a non-steroidal anti-inflammation drug.

13. The S/O type pharmaceutical preparation according to any one of claims 1 to 11, wherein the low-molecule medicine is one or more than one selected from a group consisting of diclofenac, indomethacin, and salts thereof.

14. An S/O/W type pharmaceutical preparation, wherein the S/O type preparation of any one of claims 1 to 13 is dissolved in an aqueous phase.

15. A method for producing an S/O type pharmaceutical preparation, comprising the steps of:
mixing a water-based solution containing a hydrophilic low-molecule medicine and a hydrophilic medicine-leakage-suppressive protein and/or a medicine-leakage-suppressive polysaccharide with an organic solvent solution containing a surfactant to prepare a W/O type emulsion;
lyophilizing the W/O type emulsion to prepare a medicine-containing complex; and
dissolving or dispersing the medicine-containing complex in an oil phase.

16. The method for producing an S/O type pharmaceutical preparation according to claim 15, wherein the hydrophilic medicine-leakage-suppressive protein and the medicine-leakage-suppressive polysaccharide have a molecular weight of not less than 10,000.

17. The method for producing an S/O type pharmaceutical preparation according to claim 15 or 16, wherein the hydrophilic medicine-leakage-suppressive protein is one or more than one selected from a group consisting of serum albumin, ovalbumin, casein, lysozyme, lipase, colipase, and globulin.

18. The method for producing an S/O type pharmaceutical preparation according to claim 15 or 16, wherein the hydrophilic medicine-leakage-suppressive protein is one or more than one selected from a group consisting of serum albumin, ovalbumin, and casein.

19. The method for producing an S/O type pharmaceutical preparation according to claim 15 or 16, wherein the medicine-leakage-suppressive polysaccharide is one or more than one selected from a group consisting of LM pectin, HM pectin, hydroxypropylmethyl cellulose phthalate, heparin, alginic acid, and carboxymethyl cellulose.

20. The method for producing an S/O type pharmaceutical preparation according to claim 15 or 16, wherein the medicine-leakage-suppressive polysaccharide is one or more than one selected from the group consisting of LM pectin, HM pectin, and hydroxypropylmethyl cellulose phthalate.

21. The method for producing an S/O type pharmaceutical preparation according to any one of claims 15 to 20, wherein a weight ratio of the hydrophilic medicine-leakage-suppressive protein and/or the medicine-leakage-suppressive polysaccharide with respect to the low-molecule medicine is in a range of 0.5 to 5.

22. The method for producing an S/O type pharmaceutical preparation according to any one of claims 15 to 21, wherein the oil phase is one or more than one selected from a group consisting of soybean oil, sesame oil, olive oil, safflower oil, sunflower oil, canola oil, and perilla oil.

23. The method for producing an S/O type pharmaceutical preparation according to any one of claims 15 to 22, wherein the surfactant is a non-ionic surfactant.

24. The method for producing an S/O type pharmaceutical preparation according to any one of claims 15 to 22, wherein the surfactant is one or more than one selected from a group consisting of glycerin fatty acid ester, polyglycerin fatty acid ester, polyoxyethylene glycerin fatty acid ester, sorbitan fatty acid ester, sucrose fatty acid ester, polyoxyethylene sorbit fatty acid ester, polyoxyethylene castor oil, and hardened castor oil.

25. The method for producing an S/O type pharmaceutical preparation according to any one of claims 15 to 22, wherein the surfactant has a HLB value of not more than 10.

26. The method for producing an S/O type pharmaceutical preparation according to any one of claims 15 to 25, wherein the low-molecule medicine is a non-steroidal anti-inflammation drug.

27. The method for producing an S/O type pharmaceutical preparation according to any one of claims 15 to 25, wherein the low-molecule medicine is one or more than one selected from a group consisting of diclofenac, indomethacin, and salts thereof.
